(19)

**Europäisches Patentamt**
**European Patent Office**
**Office européen des brevets**

(11) **EP 4 477 139 A1**

(12) **EUROPEAN PATENT APPLICATION**
published in accordance with Art. 153(4) EPC

(43) Date of publication:
**18.12.2024 Bulletin 2024/51**

(21) Application number: **23753186.8**

(22) Date of filing: **09.02.2023**

(51) International Patent Classification (IPC):
**A61B 5/00** (2006.01)        **A61B 5/055** (2006.01)
**G16H 50/20** (2018.01)        **G06T 7/11** (2017.01)
**G06T 7/00** (2017.01)

(52) Cooperative Patent Classification (CPC):
**A61B 5/00; A61B 5/055; G06T 7/00; G06T 7/11;
G16H 50/20**

(86) International application number:
**PCT/KR2023/001929**

(87) International publication number:
**WO 2023/153839 (17.08.2023 Gazette 2023/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.02.2022 KR 20220017193
28.10.2022 KR 20220141115**

(71) Applicant: **Samsung Life Public Welfare
Foundation
Seoul 04348 (KR)**

(72) Inventors:
• **SEO, Sang Won**
  **Seoul 06351 (KR)**
• **JANG, Hye Min**
  **Seoul 06351 (KR)**
• **PARK, Chae Jung**
  **Seoul 06351 (KR)**
• **GU, Yuna**
  **Seoul 06351 (KR)**

(74) Representative: **Ostertag & Partner Patentanwälte
mbB
Azenbergstraße 35
70174 Stuttgart (DE)**

(54) **DEMENTIA INFORMATION CALCULATION METHOD AND ANALYSIS DEVICE USING
TWO-DIMENSIONAL MRI**

(57)    This dementia information calculation method using two-dimensional (2D) magnetic resonance imaging (MRI) comprises the steps of: receiving, by an analysis device, inputs of 2D MRI slices of a subject; extracting, by the analysis device, regions of interest by inputting the 2D MRI slices to a segmentation model; predicting, by the analysis device, a cortical thickness of at least one region of the regions of interest and a volume of at least one region of the regions of interest, by inputting pixel information about the regions of interest to a pre-trained first learning model; and calculating, by the analysis device, dementia information about the subject by inputting the cortical thickness of the at least one region and the volume of the at least one region to a pre-trained second learning model.

**FIG. 1**

100

EP 4 477 139 A1

## Description

[Technical Field]

The following description relates a technique for calculating dementia-related information using two-dimensional magnetic resonance imaging (MRI).

[Background Art]

Dementia is a syndrome that causes loss of cognitive functions such as memory, language, and judgment. There are various types of dementia, and Alzheimer's disease is the most common form of dementia.

Brain images such as magnetic resonance imaging (MRI) and positron emission tomography (PET) are used to diagnose dementia. Typically, a cortical thickness is measured as a factor related to dementia.

[Disclosure]

[Technical Problem]

Conventionally, brain images such as three-dimensional (3D) magnetic resonance imaging (MRI) and positron emission tomography-computed tomography (PET-CT) were used for early dementia diagnosis. However, the 3D MRI requires high-end MRI equipment and requires a lot of time and cost. Furthermore, two-dimensional (2D) MRI scanners used in general health examination centers or primary hospitals generate approximately 20 images, which make it difficult to provide an accurate diagnosis.

The technology described below provides a technique for calculating dementia-related information or predicting dementia using a small number of 2D MRI images.

[Technical Solution]

In one general aspect, there is provided a dementia information calculation method using two-dimensional magnetic resonance imaging (2D MRI) includes: receiving, by an analysis device, inputs of 2D MRI slices of a subject; inputting, by the analysis device, the 2D MRI slices to a segmentation model to extract regions of interest (ROIs); inputting, by the analysis device, pixel information about the ROIs to a pre-trained first learning model to predict a cortical thickness of at least one region of the ROIs and a volume of at least one region of the ROIs; and inputting, by the analysis device, the cortical thickness of the at least one region and the volume of the at least one region to a pre-trained second learning model to output dementia information about the subject.

In another aspect, there is provided a dementia information calculation method using 2D MRI includes: receiving, by an analysis device, inputs of 2D MRI slices of a subject; inputting, by the analysis device, the 2D MRI slices to a segmentation model to ROIs; inputting, by the analysis device, pixel information of the ROIs to a pre-trained first learning model to predict a volume of at least one region of the ROIs; and inputting, by the analysis device, the volume of the at least one region to a pre-trained second learning model to output dementia information about the subject.

The ROIs include at least one of frontal gray matter, temporal gray matter, parietal gray matter, occipital gray matter, lateral ventricle (LV), hippocampus, and extracerebral cerebrospinal fluid.

[Advantageous Effects]

The following description provides dementia-related information using a learning model that analyzes a small number of two-dimensional magnetic resonance imaging (2D MRI) images. The following description provides information useful for dementia diagnosis based on brain MRI calculated by a low-end 2D MRI scanner.

[Description of Drawings]

FIG. 1 is an example of the entire process of calculating subject's dementia information using two-dimensional brain magnetic resonance imaging (2D brain MRI).

FIG. 2 is an example of a process of training a segmentation model that extracts a region of interest from a brain image.

FIG. 3 is an example of a process of training a learning model that predicts a cortical thickness and a volume of a brain region based on regions of interest.

FIG. 4 is an example of the process of training the learning model that outputs dementia information based on the volume of the brain region.

FIG. 5 is an example of the process of training the learning model that outputs the dementia information from the cortical thickness and the volume of the brain region.

FIG. 6 is an example of an analysis device that outputs the dementia information about a subject using the brain image.

[Exemplary embodiments of invention]

A technology to be described below may be variously modified and have several exemplary embodiments. Therefore, specific exemplary embodiments of the present disclosure will be illustrated in the accompanying drawings and be described in detail. However, it is to be understood that the technologies described below are not limited to a specific embodiment, but includes all modifications, equivalents, and substitutions without departing from the scope and spirit of the technologies described below.

Terms such as "first," "second," "A," "B," and the like, may be used to describe various components, but the components are not to be interpreted to be limited to the terms and are used only for distinguishing one component from other components. For example, a first component may be named a second component and the second component may also be similarly named the first component, without departing from the scope of the technologies described below. A term 'and/or' includes a combination of multiple related described items or any one of the plurality of related described items.

It should be understood that the singular expression include the plural expression unless the context clearly indicates otherwise, and it will be further understood that the terms "comprises" or "have" used in this specification, specify the presence of stated features, numerals, steps, operations, components, parts, or a combination thereof, but do not preclude the presence or addition of one or more other features, numerals, steps, operations, components, parts, or a combination thereof.

Prior to the detailed description of the drawings, it is intended to clarify that the components in this specification is only distinguished by the main functions of each component. That is, two or more components to be described below may be combined into one component, or one component may be divided into two or more for each subdivided function. In addition, each of the constituent parts to be described below may additionally perform some or all of the functions of other constituent parts in addition to the main functions of the constituent parts, and some of the main functions of the constituent parts may be performed exclusively by other components.

In addition, in performing the method or the operation method, each of the processes constituting the method may occur differently from the specified order unless a specific order is explicitly described in context. That is, the respective steps may be performed in the same sequence as the described sequence, be performed at substantially the same time, or be performed in an opposite sequence to the described sequence.

Hereinafter, dementia includes Alzheimer's dementia.

The technology described below is a technology that outputs dementia information from two-dimensional brain magnetic resonance imaging (2D brain MRI). The technology described below includes technology for diagnosing or predicting dementia using dementia information extracted from the 2D brain MRI.

The dementia information refers to information for dementia diagnosis or dementia prediction. For example, the dementia information includes whether the subject has dementia, whether a subject is at high risk for dementia (possibility of developing dementia in the future), a dementia-related index, etc. The dementia-related indices may include various scores that have been studied as being significant in diagnosing or predicting dementia, and a degree of brain atrophy.

Hereinafter, a device that analyzes brain MRI and outputs dementia information about a subject is referred to as an analysis device. The analysis device may be a computer device, such as a personal computer (PC), a smart device, a network server, a data processing-only chipset, etc.

The analysis device may predict dementia based on brain images using multiple learning models. The learning model is a machine learning model. Accordingly, a classification model may be any one of various types of models. For example, the learning model may be a model implemented in any one of a decision tree, a random forest (RF), a K-nearest neighbor (KNN), a naive Bayes, a support vector machine (SVM), a deep neural network (DNN), a regression model, and the like. There are various types of DNN models. For example, the DNN includes a segmentation model that extracts a region of interest (ROI) from an image, a classification model that performs classification based on features of the image, etc.

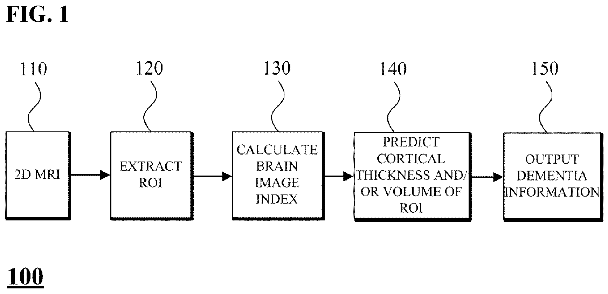

FIG. 1 is an example of the entire process 100 of calculating dementia information about a subject using 2D brain MRI.

The analysis device may output the dementia information about the subject based on a small number of 2D MRI slices. In this case, the number of 2D MRI slices may be the number calculated by a general 2D MRI scanner. A researcher constructed a learning model using 20 2D MRI slices. Therefore, for convenience of description below, it is assumed that the number of 2D MRI slices is 20.

The analysis device receives the 2D brain MRI of the subject (110). For example, the analysis device may receive 20 2D MRI slices of the subject.

The analysis device extracts the ROI from the received 2D MRI slices (120). The analysis device may extract multiple ROIs set using a pre-learned segmentation model. The ROIs may be regions for calculating a volume of a specific region. Furthermore, the ROIs may include regions for calculating a cortical thickness.

The ROIs may include at least one of frontal gray matter, temporal gray matter, parietal gray matter, occipital gray matter, lateral ventricle (LV), hippocampus, and extracerebral cerebrospinal fluid.

The analysis device may calculate a certain brain image index using the extracted ROIs (130).

The brain image index may include the number of pixels in the extracted ROI. In addition, the brain image index may include the total brain size that can be calculated from the sizes of the regions of the brain image. The brain size may be calculated by summing the sizes of extracerebral cerebrospinal fluid, the above-described seven ROIs, and white matter of the ROIs. Meanwhile, since various methodologies for determining the brain size have been studied, the analysis device may calculate the brain size of the subject using one of various image processing technologies.

Meanwhile, the analysis device may normalize the ROI based on a preset size. For example, the analysis device may calculate the entire brain size and normalize the size of the ROI based on a certain level. The analysis device may calculate the brain image index equal to the number of pixels based on the normalized ROI(s).

The analysis device may estimate the volume of the brain region using the pre-trained learning model (140). Alternatively, the analysis device may estimate the cortical thickness and the volume of the brain region using the pre-trained learning model (140).

The analysis device may input the brain image index to the learning model to predict the cortical thickness of the ROI. The analysis device may predict the cortical thickness in each ROI using multiple learning models. For example, the analysis device may calculate the cortical thickness of each region using individual learning models for the frontal, temporal, parietal, and occipital.

In addition, the analysis device may predict the volume of the brain region using a separate learning model. The analysis device may input the brain image index to the learning model to predict the volume of the brain region. The analysis device may also predict the volume in each ROI using multiple learning models. For example, the analysis device may individually use the learning model for the LV, the learning model for the hippocampus, and the learning model for the extracerebral cerebrospinal fluid to calculate the volumes of each region.

Meanwhile, the analysis device may calculate the cortical thickness or the volume of the brain region using the clinical information (gender, age, etc.) of the subject in addition to the brain image. In this case, each learning model should be pre-trained to calculate the cortical thickness or the volume of the brain region using clinical information in addition to the brain MRI.

The analysis device may output the dementia information about the subject based on the cortical thickness and the volume of the ROI(s) (150). Alternatively, the analysis device may output the dementia information about the subject based only on the volume of the ROI(s) (150).

The process of calculating the dementia information in FIG. 1 uses multiple learning models to output the dementia information about the subject. The researcher constructed multiple learning models for the above-described process and verified the performance of individual models and the performance of dementia information calculation. Hereinafter, the process of constructing, by the researcher, the learning models used to output the dementia information about the subject will be described.

[Table 1]

| | Entire population | | ADD (dementia patient) | | NC(normal) | |
|---|---|---|---|---|---|---|
| | Mean | Standard deviation (%) | Mean | Standard deviation (%) | Mean | Standard deviation (%) |
| Subjects (name) | 1,120 | (100.0%) | 529 | (47.2%) | 591 | (52.8%) |
| Female (name) | 659 | (58.8%) | 306 | (57.8%) | 353 | (59.7%) |
| Age (years) | 69.0 | 10.9 | 69.9 | 10.2 | 68.2 | 11.5 |
| Education (years) | 11.7 | 4.8 | 11.3 | 4.9 | 12.1 | 4.7 |
| Frontal cortical thickness (mm) | 3.087 | 0.152 | 3.007 | 0.154 | 3.158 | 0.108 |
| Parietal cortical thickness (mm) | 2.984 | 0.192 | 2.877 | 0.200 | 3.080 | 0.120 |

(continued)

| | Entire population | | ADD (dementia patient) | | NC(normal) | |
|---|---|---|---|---|---|---|
| | Mean | Standard deviation (%) | Mean | Standard deviation (%) | Mean | Standard deviation (%) |
| Temporal cortical thickness (mm) | 3.186 | 0.189 | 3.068 | 0.185 | 3.291 | 0.117 |
| Occipital cortical thickness (mm) | 2.897 | 0.204 | 2.793 | 0.186 | 2.991 | 0.171 |
| Lateral ventricle volume (mm$^3$) | 36123.93346 | 20197.7947 | 44637.0 | 20985.2 | 28507.7 | 16056.7 |
| Hippocampus volume (mm$^3$) | 2,627 | 632 | 2,177 | 523 | 3,031 | 411 |
| Frontal extracerebral CSF volume (mm$^3$) | 62450.29197 | 21740.67107 | 72456.38 | 21692.64 | 52262.27 | 16416 |
| Temporal extracerebral CSF volume (mm$^3$) | 37218.44005 | 12610.11551 | 42089 | 12551.5 | 32259.33 | 10580.77 |
| Parietal extracerebral CSF volume (mm$^3$) | 36737.31928 | 12420.93104 | 42753.81 | 12495.3 | 30611.44 | 8839.807 |
| Occipital extracerebral CSF volume (mm$^3$) | 15064.27969 | 4565.302592 | 16663.86 | 4881.548 | 13435.62 | 3545.16 |
| Anterior lateral ventricle (LV) volume (mm$^3$) | 17740.63456 | 9031.29889 | 9074.209 | 9074.209 | 14515.32 | 7762.385 |
| Posterior LV volume (mm$^3$) | 20459.65506 | 12092.68325 | 12581.57 | 12581.57 | 15613.22 | 9357.293 |

The researcher constructed a segmentation model using data of 980 people among population data. The data of 980 people is 3D MRI data generated using a 3.0 T MRI scanner (Philips 3.0T Achieva). The researcher performed structural image analysis using a 3D segmentation mask of the CIVET pipeline. In the CIVET, the cortical thickness is calculated as a Euclidean distance between inner and outer surfaces of a cerebral cortex. The ROIs include (i) frontal, temporal, parietal, and occipital gray matters, (ii) LV (iii) hippocampus, and (iv) extracerebral cerebrospinal fluid. The researcher used the ROIs extracted from the data of 980 people as ground truth for training the segmentation model.

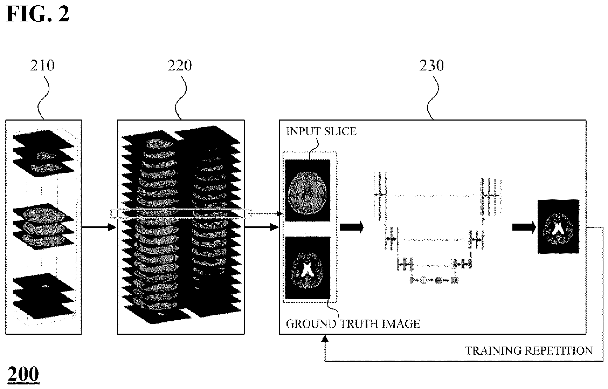

FIG. 2 is an example of a process 200 to train the segmentation model to extract the ROI from the brain image. Hereinafter, the process of training the model will be described as being performed by a learning device. The learning device refers to a computer device capable of processing image data and performing a process of training a machine learning model.

The learning device trains the segmentation model using MRI images of multiple subjects. However, FIG. 2 illustrates the process of training the segmentation model using MRI of one subject as an example.

The learning device receives 3D MRI of a specific subject belonging to the above-described population (210).

The learning device prepares a training data set (220). The training data set is composed of input slices (original slices) by multiple slices and a ground truth image for each slice. The learning device selects 20 slices from 480 slices of the 3D MRI. The learning device may select slices that match 20 slices acquired from the 2D MRI scanner among 480 slices. In addition, the learning device receives the ground truth images for the 20 selected slices. The ground truth image is an image dividing the ROIs for the input slice. As the ground truth image, an image dividing and marking (annotating) ROIs by a medical imaging expert may be used.

The learning device trains the segmentation model using the training data (230). The learning device may train the segmentation model using input slices and ground truth images for 20 slices. The segmentation model is trained to receive the input slices and extract (classify) ROIs from the ground truth image.

The segmentation model may be a semantic segmentation model. For example, the segmentation model may be a fully convolutional network (FCN)-based model such as U-net. The researcher extracted the ROI from the 2D MRI slice using a U-net structured segmentation model.

The segmentation model may be prepared in advance for each ROI. As described above, the ROIs may include at least one of the frontal gray matter, the temporal gray matter, the parietal gray matter, the occipital gray matter, the lateral ventricle (LV), the hippocampus, and the extracerebral cerebrospinal fluid region.

Alternatively, the segmentation model may be trained to extract multiple ROIs. The researcher performed curriculum learning to accurately extract various ROIs with different characteristics. The curriculum learning is a learning strategy that imitates the human learning process to first train data with an easy difficulty level, and then train data with progressively higher difficulty.

The researcher constructed the segmentation model according to five training orders below and verified its performance.

[Table 2]

| No. | Training order |
|---|---|
| 1 | All regions of interest (All ROIs) |
| 2 | Hippocampus→hippocampus and lateral ventricle (LV)→ all ROIs |
| 3 | Hippocampus and LV→all regions of interest |
| 4 | LV→hippocampus and LV→all ROIs |
| 5 | LV→all ROIs |

The performance of the segmentation model was measured by an intersection over union (IoU) and a dice similarity coefficient (DSC) that are calculated based on the ground truth and the ROI extracted by the segmentation model. The IoU and DSC may be expressed as Equation 1 and Equation 2 below, respectively.

$$[\text{Equation 1}]$$

$$IoU = A_{overlap} / (A_{GT} + A_{pred} - A_{overlap})$$

$$[\text{Equation 2}]$$

$$DSC = 2 * A_{overlap} / (A_{GT} + A_{pred})$$

In the above Equations, $A_{GT}$ is the ROI which is the ground truth, $A_{pred}$ is the ROI (predicted region) divided by the segmentation model, and $A_{overlap}$ is an overlapping region between the $A_{GT}$ and $A_{pred}$.

The segmentation model showed the IoU and DSC as shown in Table 3 below for six ROIs below. The segmentation model showed the highest performance in the LV extraction.

[Table 3]

| Region of interest | IoU | DSC |
|---|---|---|
| Frontal | 0.71356 | 0.83168 |
| Temporal | 0.67835 | 0.80696 |
| Parietal | 0.64923 | 0.7857 |
| Occipital | 0.57339 | 0.72749 |
| Lateral ventricle | 0.87904 | 0.93524 |
| Hippocampus | 0.6961 | 0.8188 |
| Mean | 0.69828 | 0.81765 |

Table 4 below shows the extraction performance of the segmentation model for the extracerebral cerebrospinal fluid. The segmentation model showed the highest performance in the LV region.

[Table 4]

| Region of interest | IoU | DSC |
|---|---|---|
| Frontal extracerebral CSF | 0.468802 | 0.620074 |
| Temporal extracerebral CSF | 0.325105 | 0.47922 |
| Parietal extracerebral CSF | 0.459181 | 0.604894 |
| Occipital extracerebral CSF | 0.293677 | 0.443401 |
| Anterior lateral ventricle (LV) extracerebral CSF | 0.771025 | 0.861737 |
| Posterior LV extracerebral CSF | 0.731659 | 0.835934 |
| Mean | 0.508241 | 0.640877 |

Table 5 below shows the results of analyzing the performance of the training order in Table 2. The learning method in which the LV was trained first showed somewhat higher performance than the learning methods in other sequences. Therefore, it can be seen that the technique of performing the curriculum learning by dividing the ROI is significant for the performance of the segmentation model.

[Table 5]

| No. | Training order | IoU |
|---|---|---|
| 1 | All regions of interest (All ROIs) | 0.653 |
| 2 | Hippocampus→hippocampus and lateral ventricle (LV)→all ROIs | 0.565 |
| 3 | Hippocampus and LV→all ROIs | 0.665 |
| 4 | LV→hippocampus and LV→all ROIs | 0.683 |
| 5 | LV→all ROIs | 0.689 |

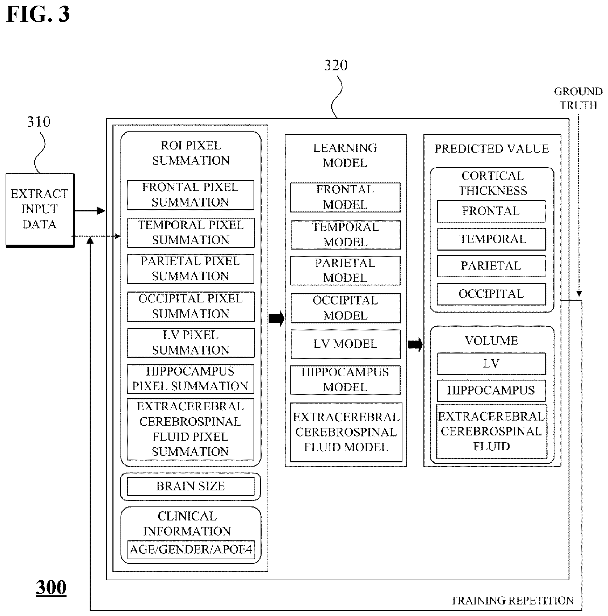

FIG. 3 is an example of a process 300 of training a learning model that predicts a cortical thickness and a volume of a brain region based on ROIs. The learning models that predict the cortical thickness may be prepared in advance for each ROI. In addition, the learning models that predict the volume of the brain region may also be prepared in advance for each ROI. In FIG. 3, the learning model may predict the cortical thickness or the volume of the brain region based on each ROI. FIG. 3 shows the learning process for each ROI.

The learning in FIG. 3 assumes that the above-described ROI has been extracted from brain MRI of a subject belonging to the population. That is, the learning device may use the ROI calculated using the segmentation model in FIG. 2.

The learning device extracts input data based on the ROIs extracted from the brain MRI (310). The input data may include brain image index and clinical information extracted from the ROIs extracted by the segmentation model.

The brain image index includes size information for each ROIs (frontal gray matter, temporal gray matter, parietal gray matter, occipital gray matter, LV, hippocampus, and extracerebral cerebrospinal fluid). The size of the ROI may be determined by the number of pixels in the corresponding ROI image. In other words, the size of the ROI may be the summed number of all pixels belonging to the region. Meanwhile, the size of the ROI may be standardized based on the overall brain size. The standardization is the process of correcting a brain size that varies for each subject to subject to a constant size.

In addition, the brain image index may further include the brain size. The brain size may be calculated by summing the sizes of the extracerebral cerebrospinal fluid, the above-described seven ROIs, and the white matter of the ROIs.

The summed number of pixels of a specific ROI may be the summed number of pixels of the ROI for each of the 20 slices. When describing based on the frontal gray matter, the summed number of pixels of frontal gray matter may be a value obtained by summing all pixels belonging to the frontal gray matter region in each of the 20 slices. It is assumed that the indices of 20 slices are 0 to 19. The summed number of pixels in the frontal gray matter may be a value obtained by summing the number of pixels belonging to the frontal gray matter region of slice 0, the number of pixels belonging to the frontal gray matter region of slice 1,..., and the number of pixels belonging to the frontal gray matter region of slice 19. Furthermore, the number of pixels in the ROI may be a value obtained by averaging the number of pixels in all slices.

The clinical information may include an age and gender of a subject. In addition, the clinical information may also include the APOE4 genotype.

The researcher used all the summed number of pixels in the ROIs, the brain size, and the clinical information as input data.

The learning device performs the process of training the learning model using the extracted input data (320). The learning device repeats the process of training the learning model using the input data extracted from the brain MRI of multiple subjects.

The learning device performs the training by inputting the extracted input data into the learning model and comparing the value (predicted value) output by the learning model with the ground truth. The learning model is trained to predict a cortical thickness of a specific ROI or a volume of a specific ROI. The ground truth is information calculated from MRI and PET-CT of the subjects belonging to the population (see Table 1).

The learning model may be constructed as an individual model depending on the information to be predicted. The learning model is a machine learning model and may be implemented as one of various types of models. The researcher used a regression model. The researcher individually constructed a model (frontal model) predicting the cortical thickness of the frontal gray matter, a model (temporal model) predicting the cortical thickness of the temporal gray matter, a model (parietal model) predicting the cortical thickness of the parietal gray matter, a model (occipital model) predicting the cortical thickness of the occipital gray matter, a model (LV model) predicting the volume of the LV, a model (hippocampus model) predicting the volume of the hippocampus, and a model (extracerebral cerebrospinal fluid model) predicting the volume of the extracerebral cerebrospinal fluid.

For example, the frontal model may receive the summed number of pixels of the frontal gray matter and the clinical information to predict the cortical thickness of the frontal region. In addition, the frontal model may receive the summed number of pixels of the frontal gray matter, the brain size, and the clinical information to predict the cortical thickness of the frontal region.

For example, the extracerebral cerebrospinal fluid model may receive the summed number of pixels of the extracerebral cerebrospinal fluid and the clinical information to predict the volume of the extracerebral cerebrospinal fluid. In addition, the extracerebral cerebrospinal fluid model may receive the summed number of pixels of the extracerebral cerebrospinal fluid, the brain size, and the clinical information to predict the volume of the extracerebral cerebrospinal fluid.

The researcher constructed a model using the brain size as the input data and a model not using the brain size as the input data to verify the performance of the models. Table 6 below is a model that evaluates the performance of the learning model constructed by the researcher. The performance index is a Pearson correlation coefficient. In Table 6, model 1 is a model that does not use the brain size, and model 2 is a model that uses the brain size as well. Depending on the ROI, it can be seen that model 1 or model 2 has slightly higher performance. Therefore, the model predicting the cortical thickness and the volume of the brain region may selectively use model 1 or model 2 depending on the ROI. However, there was no significant difference between model 1 and model 2 in performance.

[Table 6]

| ROI | Model 1 | Model 2 |
| --- | --- | --- |
| Frontal gray matter | 0.774 | 0.779 |
| Temporal gray matter | 0.786 | 0.788 |
| Parietal gray matter | 0.801 | 0.799 |
| Occipital gray matter | 0.649 | 0.638 |
| Lateral ventricle | 0.880 | 0.869 |
| Hippocampus | 0.663 | 0.667 |

The researcher verified the performance of the learning model that predicts the cortical thickness of the ROI or the volume of the ROI. The researcher used the learning model to compare the predicted value based on the 2D MRI with the ground truth value measured from the 3D MRI. The comparison results are shown in Table 7 below. The cortical thickness and volume predicted by the learning model showed significant correlation with the actual ground truth values. In particular, the volume showed high correlation.

The researcher verified the performance of the learning model that predicts the cortical thickness of the ROI or the volume of the ROI. The researcher used the learning model to compare the predicted value based on the 2D MRI with the ground truth value measured from the 3D MRI. The comparison results are shown in Table 7 below. The cortical thickness and volume predicted by the learning model showed significant correlation with the actual ground truth values. In particular, the volume showed high correlation.

[Table 7]

| Division | ROI | Correlation (Pearson r) | r2 | slope |
|---|---|---|---|---|
| Cortical thickness | Frontal gray matter | 0.779 | 0.607 | 0.394 |
| Cortical thickness | Temporal gray matter | 0.788 | 0.621 | 0.396 |
| Cortical thickness | Parietal gray matter | 0.801 | 0.641 | 0.519 |
| Cortical thickness | Occipital gray matter | 0.649 | 0.394 | 0.285 |
| Volume | Lateral ventricle | 0.879 | 0.754 | 0.757 |
| Volume | Hippocampus | 0.667 | 0.445 | 0.266 |
| Volume | Extracerebral cerebrospinal fluid | 0.873 | 0.762 | 0.608 |

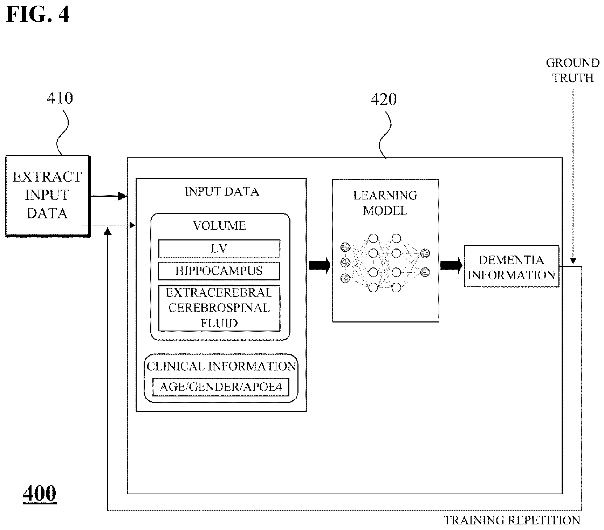

FIG. 4 is an example of the process 400 of training the learning model that outputs dementia information based on the volume of the brain region. Ultimately, the learning model that outputs the dementia information may be implemented as one of various machine learning model types. The researcher implemented a model that outputs the dementia information using a deep learning model.

The learning in FIG. 4 assumes that a cortical thickness, a volume of a specific brain region, and clinical information for subjects belonging to the population have been acquired. For example, the learning device may use a volume of a specific brain region for each region of a subject calculated using the learning model of FIG. 3.

The learning device may extract input data necessary for learning (410). The learning device extracts a volume of a specific brain region extracted from 2D brain MRI of subjects belonging to the population. The volume of the brain region may include the volumes of the LV, the hippocampus, and the extracerebral cerebrospinal fluid. Additionally, the learning device acquires clinical information of subjects. The clinical information may include at least one of the age, gender, and APOE4 genotype. The training data may include the volume of the specific brain region calculated using the learning model in FIG. 3. Alternatively, the training data may include the cortical thickness and the volume of the specific brain region calculated from brain images of actual subjects. The researcher used the cortical thickness and the volume of the specific brain region calculated from the 3D MRI analysis results of actual subjects as the training data.

The learning device performs the process of training the learning model using the extracted input data (420). The learning device repeats the process of training the learning model using the input data extracted from multiple subjects.

The learning device performs the training by inputting the extracted input data into the learning model and comparing the value (predicted value) output by the learning model with the ground truth. The learning model is trained to output the dementia information about the subject. For example, the learning model may output binary classification results regarding whether the subject has dementia. In this case, the ground truth is information about whether the subjects belonging to the population have dementia (see Table 1).

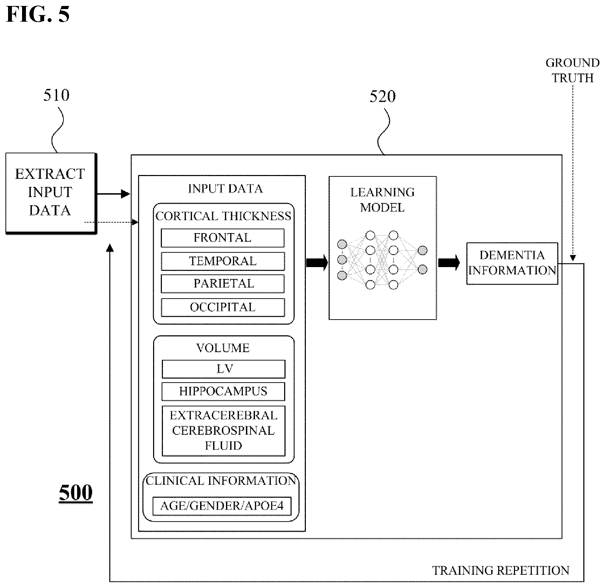

FIG. 5 is an example of a process 500 of training the learning model that outputs the dementia information from the cortical thickness and the volume of the brain region. Ultimately, the learning model that outputs the dementia information may be implemented as one of various machine learning model types. The researcher implemented a model that outputs the dementia information using a deep learning model.

The learning in FIG. 5 assumes that a cortical thickness, a volume of a specific brain region, and clinical information for subjects belonging to the population have been acquired. For example, the learning device may use a cortical thickness and a volume of a specific brain region for each region of a subject calculated using the learning model of FIG. 3.

The learning device may extract input data necessary for learning (510). The learning device extracts the cortical thickness and the volume of the specific brain region extracted from the brain MRI of the subjects belonging to the population. The cortical thickness may include at least one of the cortical thicknesses of the frontal gray matter, temporal gray matter, parietal gray matter, and occipital gray matter. The volume of the brain region may include at least one of the volumes of the LV, the hippocampus, and the extracerebral cerebrospinal fluid.

Additionally, the learning device acquires the clinical information of subjects. The clinical information may include at least one of the age, gender, and APOE4 genotype. The training data may include the cortical thickness and the volume of the specific brain region calculated using the learning model in FIG. 3. Alternatively, the training data may include the cortical thickness and the volume of the specific brain region calculated from brain images of actual subjects. The researcher used the cortical thickness and the volume of the specific brain region calculated from the 3D MRI analysis results of actual subjects as the training data.

The learning device performs the process of training the learning model using the extracted input data (520). The learning device repeats the process of training the learning model using the input data extracted from multiple subjects.

The learning device performs the training by inputting the extracted input data into the learning model and comparing the value (predicted value) output by the learning model with the ground truth. The learning model is trained to output the dementia information about the subject. For example, the learning model may output binary classification results regarding whether the subject has dementia. In this case, the ground truth is information about whether the subjects belonging to the population have dementia (see Table 1).

The researcher constructed multiple learning models using different input data sets. The researcher used data from 924 people (80%) of the population as training data, and data from 196 people (20%) as verification data. The researcher largely divided model groups using different ROIs and separately constructed models using various input data in each group. The multiple learning models constructed by the researcher are shown in Table 8 below. Table 8 shows model groups using four different ROIs and the training data used to build different models in each group. Table 8 below is a model that predicts whether the subject has dementia (dementia or normal). That is, the learning model in FIG. 4 corresponds to a model that binary-classifies whether the subject has dementia or is normal.

[Table 8]

| Model type | | Training data (input data) |
|---|---|---|
| 6ROI (F/T/P/O/L/H) | Learning model 1 | Cortical thickness of frontal, cortical thickness of temporal, cortical thickness of parietal, cortical thickness of occipital, volume of lateral ventricle (LV), and volume of hippocampus |
| | Learning model 2 | Learning model 1 + clinical information (age, gender) |
| | Learning model 3 | Learning model 2 + clinical information (APOE4) |
| 7ROI (F/T/P/O/L/H/E) | Learning model 4 | Cortical thickness of frontal, cortical thickness of temporal, cortical thickness of parietal, cortical thickness of occipital, volume of LV, volume of hippocampus, and volume of extracerebral cerebrospinal fluid |
| | Learning model 5 | Learning model 4 + clinical information (age, gender) |
| | Learning model 6 | Learning model 5 + clinical information (APOE4) |
| 3ROI (L/H/E) | Learning model 7 | Volume of LV, volume of hippocampus, and volume of extracerebral cerebrospinal fluid |
| | Learning model 8 | Learning model 7 + clinical information (age, gender) |
| | Learning model 9 | Learning model 8 + clinical information (APOE4) |
| 4ROI (F/T/P/O) | Learning model 10 | Cortical thickness of frontal, cortical thickness of temporal, cortical thickness of parietal, cortical thickness of occipital |
| | Learning model 11 | Learning model 10 + clinical information (age, gender) |
| | Learning model 12 | Learning model 11 + clinical information (APOE4) |

In Table 8, the ROI is abbreviated. F refers to frontal, T refers to temporal, P refers to parietal, O refers to occipital, L refers to lateral ventricle, H refers to hippocampus, and E refers to extracerebral cerebrospinal fluid. Meanwhile, unlike Table 8, the learning model may be a model that uses the thickness and/or volume of the region of some (at least one) of the above-described ROIs as the input data. In other words, various types of models may be constructed depending on which region of the ROIs is used for the learning model.

The researcher verified the constructed learning model. Meanwhile, during the verification process, the researcher used the cortical thickness and the volume of the brain region predicted by the learning model in FIG. 3 as the input data for the learning model in FIG. 4. The researcher performed 10-fold cross-verification. The verification results are shown in Table 9 below. The verification compared the results predicted by the learning model with the ground truth. The performance index reflects an area under the receiver operating characteristic curve (AUC) and area under the precision-recall curve (AUPRC).

The researcher compared the performance of the conventional 3D MRI-based classification model and the above-described 2D MRI-based learning model. The conventional 3D MRI-based classification model used the previously studied model (Rebsamen, M., et al., Direct Cortical Thickness Estimation Using Deep Learning-Based Anatomy Segmentation and Cortex Parcellation. Human Brain Mapping, 2020. 41(17): p. 4804-4814.).

Looking at the results in Table 9, the performance difference between the 3D MRI-based model and the 2D MRI-based model is not significant. Therefore, it can be said that the 2D MRI-based model developed by the researcher is also sufficiently significant in predicting dementia. In addition, a model (learning models 10 to 12) using only the cortical thickness of the ROIs extracted from the 2D MRI, a model (learning models 7 to 9) using only the volume of ROIs, and a model (learning models 1 to 6) using the cortical thickness and volume of ROIs all showed a significant level of performance. Compared to the model (learning model 10 to 12) using only the cortical thickness, other models showed slightly higher performance.

[Table 9]

| Model type | Conventional 3D MRI-based learning model | | 2D MRI-based learning model | |
|---|---|---|---|---|
| | AUC | AUPRC | AUC | AUPRC |
| Learning model 1 | 0.938 | 0.933 | 0.913 | 0.883 |
| Learning model 2 | 0.936 | 0.935 | 0.922 | 0.900 |
| Learning model 3 | 0.935 | 0.945 | 0.916 | 0.878 |
| Learning model 4 | 0.928 | 0.921 | 0.915 | 0.889 |
| Learning model 5 | 0.941 | 0.943 | 0.918 | 0.902 |
| Learning model 6 | 0.947 | 0.949 | 0.927 | 0.904 |
| Learning model 7 | 0.909 | 0.904 | 0.841 | 0.754 |
| Learning model 8 | 0.929 | 0.928 | 0.901 | 0.863 |
| Learning model 9 | 0.935 | 0.935 | 0.906 | 0.868 |
| Learning model 10 | 0.845 | 0.872 | 0.869 | 0.821 |
| Learning model 11 | 0.863 | 0.885 | 0.877 | 0.828 |
| Learning model 12 | 0.892 | 0.902 | 0.876 | 0.825 |

In addition, the researcher also constructed a model to calculate an index related to dementia in previous studies. The researcher used data from 924 people (80%) of the population as training data, and data from 196 people (20%) as validation data. The researcher constructed a model that receives the cortical thickness and/or volume of the ROI calculated using the learning model of FIG. 3 described above to calculate a certain index. In FIG. 4, the learning model corresponds to a model that calculates a certain index (score).

In previous studies, a W-score corresponds to an index that determines brain atrophy information (see Renaud La Joie et al., Region-Specific Hierarchy Between Atrophy, Hypometabolism, and β-amyloid (Aβ) load in Alzheimer's Disease Dementia, J. Neurosci. 2012 Nov 14;32(46): 16265-73.) Subjects with a W-score of 1.65 or higher may be determined to have brain atrophy. The researcher constructed a learning model that calculates the W-score. Table 10 below shows the correlation between the W-score of the learning model constructed by the researcher and the W-score calculated using the 3D MRI of the same subject. Table 10 compares the results of calculating scores for each ROI. In Table 10, model 1 uses the age and gender as score correction variables, and model 2 uses the age, gender, and education level as score correction variables.

[Table 10]

| | Entire area | Frontal | Temporal | Parietal | Occipital | Hippocampus | Lateral ventricle | Extracerebral cerebrospinal fluid |
|---|---|---|---|---|---|---|---|---|
| Model 1 | 0.826 | 0.759 | 0.768 | 0.819 | 0.639 | 0.721 | 0.918 | 0.774 |
| Model2 | 0.834 | 0.762 | 0.783 | 0.821 | 0.651 | 0.706 | 0.916 | 0.764 |

In previous studies, the AD-score was an index for determining dementia (see Jin San Lee et al., Machine Learning-based Individual Assessment of Cortical Atrophy Pattern in Alzheimer's Disease Spectrum: Development of the Classifier and Longitudinal Evaluation, J Neurosci. SCIENTIFIC REPORTS, Vol.8(1): 4161, 2018).

The researcher constructed a learning model that calculates the AD-score using the data from the above-described population. In other words, in FIG. 4, this corresponds to the case where the learning model calculates the AD-score. The researcher verified the constructed model using test data from 196 pages. As a result of the verification, the learning model that calculates the AD-score showed high performance with AUC = 0.92 and Accuracy = 0.854.

Furthermore, the researcher calculated the AD-score using the learning model that calculates the W-score described above. The researcher used the learning model to derive the AD-score using the W-score for the above-described seven ROIs as the input value. The researcher analyzed the correlation between the AD-score calculated using the learning model and the AD-score according to previous research methods. Table 11 below shows the performance of the learning model that calculates the W-score. Model 1 uses the age and gender as score correction variables, and model 2 uses the age, gender, and education level as score correction variables. Looking at Table 11, it can be seen that even when using the model that calculates the W-score, there is a significant correlation with the ground truth value.

[Table 11]

| Model division | PCA | AD score correlation r | r2 | slope |
|---|---|---|---|---|
| Model 1 | 2 components | 0.897 | 0.805 | 1.009 |
| | 3 components | 0.895 | 0.794 | 1.011 |
| Model 2 | 2 components | 0.908 | 0.825 | 0.825 |
| | 3 components | 0.911 | 0.830 | 0.824 |

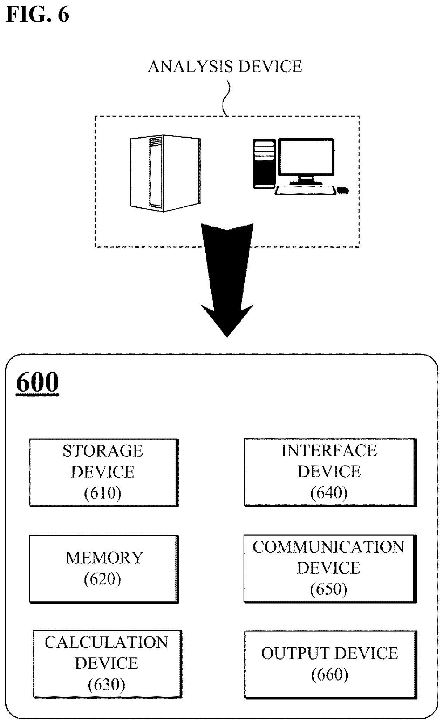

FIG. 6 is an example of an analysis device 600 that outputs the dementia information about the subject using the brain image. The analysis device 600 may be physically implemented in various forms. For example, the analysis device 600 may have the form of a computer device such as a PC, a smart device, a server of a network, a data processing-only chipset, or the like. Meanwhile, the analysis device 600 may be connected to brain image equipment or may be an integrated device.

The analysis device 600 may include a storage device 610, a memory 620, a calculation device 630, an interface device 640, a communication device 650, and an output device 660.

The storage device 610 may store the 2D MRI of the subject generated by the medical imaging equipment.

The storage device 610 may store the clinical information of the subject. The clinical information may include at least one of age, gender, education level, and APOE4 genotype.

The storage device 610 may store the segmentation model for extracting the ROI from the brain image (2D MRI slice). The segmentation model is a pre-trained model.

The storage device 610 may store a learning model that predicts the cortical thickness and/or the volume of the specific brain region based on the ROI information and the clinical information (age and gender). The learning model that predicts the cortical thickness and/or the volume of the specific brain region is called a first learning model. As described above, the first learning model uses the summed number of pixels of the ROI (at least one of frontal gray matter, temporal gray matter, parietal gray matter, occipital gray matter, LV, hippocampus, and extracerebral cerebrospinal fluid) and the clinical information as the input data. Furthermore, the first learning model may further use the brain size as the input data. Therefore, the first learning model may be prepared in advance by being divided into the model that does not use the brain size and the model that uses the brain size. In addition, the first learning model may be prepared in advance for each ROI.

The storage device 610 may store the learning model that outputs the dementia information based on the cortical thickness and/or the volume of the specific brain region. Ultimately, the learning model that predicts dementia is called a second learning model. As described above, the second learning model may be implemented as various models depending on the type of input data. The second learning model may be any one of (i) a model using only the cortical thickness and the volume of the specific brain region as the input data, (ii) a model using the cortical thickness, the volume of the specific brain region, and the clinical information (age, gender and APEO4) as the input data, (iii) a model using only the cortical thickness of the specific ROIs as the input data, (iv) a model using the cortical thickness of the specific ROIs and the clinical information (age, gender, and APEO4) as the input data, (v) a model using only the volume of the specific ROIs as the input data, and (vi) a model using the volume of the specific ROI and the clinical information (age, gender, and APEO4) as the input data. The second learning model may be any one of the models described in Table 7.

The dementia information may be any one of information such as a prediction result of whether a subject has dementia, a prediction result of whether a subject is at high risk for dementia, or a dementia-related index (brain atrophy information, W-score, AD-score, etc.).

The memory 620 may store data, information, and the like generated in the process in which the analysis device 600 outputs the dementia information from the brain image.

The interface device 640 is a device that receives predetermined commands and data from the outside. The interface device 640 may receive the 2D MRI and clinical information of the subject from the physically connected input device or the external storage device. The number of input 2D MRI slices is about 20. The interface device 640 may transmit the dementia information predicted based on the 2D MRI to an external object.

The communication device 650 refers to a configuration for receiving and transmitting predetermined information through a wired or wireless network. The communication device 650 may receive the 2D MRI and clinical information of the subject from the external object. The number of 2D MRI slices is about 20. The communication device 650 may transmit the dementia information predicted based on the 2D MRI to the external object such as a user terminal.

The interface device 640 may be a device that internally receives data received from the communication device 650.

The output device 660 is a device that outputs predetermined information. The output device 660 may output an interface required for the data processing process, the brain image, the ROI divided from the brain image, the dementia-related index calculated based on the ROI, the dementia information, etc.

The calculation device 630 may regularly preprocess the brain image (2D MRI) of the subject. The calculation device 630 may generate a mask for dividing the entire brain region through the data preprocessing process. The calculation device 630 may divide the entire brain region from the brain image using the mask for the entire brain region.

The calculation device 630 may regularly normalize the size or resolution of the 2D MRI of the subject.

The calculation device 630 may extract the ROI by inputting the 2D MRI slice of the subject to the trained segmentation model. The ROIs include at least one of the frontal gray matter, the temporal gray matter, the parietal gray matter, the occipital gray matter, the LV, the hippocampus, and the extracerebral cerebrospinal fluid.

The calculation device 630 may extract the size information about the ROIs. The calculation device 630 may calculate the summed number of pixels for each ROI. The calculation device 630 may sum the number of pixels identified in all the 2D slices for each ROI (e.g., frontal) to calculate the summed number of pixels.

The calculation device 630 may calculate the entire brain size based on the size of the ROI and other regions in the brain MRI. The brain size may be determined by summing the sizes of the above-described seven ROIs, and the white matter of the ROIs.

The calculation device 630 may input the summed number of pixels of the ROI and the clinical information (at least one of age, gender, and APEO4 genotype) to the first learning model to predict the cortical thickness and volume of the ROI. The calculation device 630 may input the summed number of pixels of the ROI and the clinical information (at least one of age, gender, and APEO4 genotype) to the individual learning models to predict the cortical thickness and volume of the ROI. In this case, the calculation device 630 may input the cortical thickness and volume of the ROI(s) predicted in the first learning model to the second learning model to output the dementia information about the subject. For example, the calculation device 630 may input the cortical thickness of the frontal gray matter, the cortical thickness of the temporal gray matter, the cortical thickness of the parietal gray matter, the cortical thickness of the occipital gray matter, the volume of the LV, the volume of the hippocampus, and the volume of the extracerebral cerebrospinal fluid to the second learning model to output the dementia information about the subject.

In addition, the calculation device 630 may input the summed number of pixels of the ROI, the brain size, and the clinical information (at least one of age, gender, and APEO4 genotype) to the first learning model to predict the volume of the ROI. The calculation device 630 may input the summed number of pixels of the ROI, the brain size, and the clinical information (at least one of age, gender, and APEO4 genotype) to the individual learning models to predict the volume of the ROI. In this case, the calculation device 630 may input the volume of the ROI(s) predicted in the second learning model to the second learning model to output the dementia information about the subject. For example, the calculation device 630 may input the volume of the LV, the volume of the hippocampus, and the volume of the extracerebral cerebrospinal fluid to the second learning model to output the dementia information about the subject.

The calculation device 630 may further input the clinical information (at least one of age, gender, and APOE4 genotype) to the second learning model in addition to the cortical thickness or the volume to output the dementia information about the subj ect.

The calculation device 630 may be a device such as a processor, an AP, or a chip embedded with a program that processes data and processes a predetermined operation.

In addition, the dementia information calculation method using 2D MRI and the dementia prediction method using 2D MRI as described above may be implemented as a program (or application) that includes an executable algorithm that may be executed on a computer. The program may be stored and provided in a transitory or non-transitory computer readable medium.

The non-transitory computer readable medium is not a medium that stores data for a while, such as a register, a cache, a memory, or the like, but means a medium that semi-permanently stores data and is readable by an apparatus. Specifically, various applications or programs described above may be provided by being stored in non-transitory readable media such as a compact disc (CD), a digital video disc (DVD), a hard disk, a Blu-ray disc, a universal serial bus (USB), a memory card, a read-only memory (ROM), a programmable read only memory (PROM), an erasable PROM (EPROM), an electrically EPROM (EEPROM), or a flash memory.

The transitory readable medium refers to various RAMs such as a static RAM (SRAM), a dynamic RAM (DRAM), a synchronous DRAM (SDRAM), a double data rate SDRAM (DDR SDRAM), an enhanced SDRAM (ESDRAM), a synclink DRAM (SLDRAM), and a direct rambus RAM (DRRAM).

The present embodiment and the drawings attached to the present specification only clearly show some of the technical ideas included in the above-described technology, and therefore, it will be apparent that all modifications and specific embodiments that can be easily inferred by those skilled in the art within the scope of the technical spirit included in the specification and drawings of the above-described technology are included in the scope of the above-described technology.

**Claims**

1. A dementia information calculation method using two-dimensional magnetic resonance imaging (2D MRI), comprising:

   receiving, by an analysis device, inputs of 2D MRI slices of a subject;
   inputting, by the analysis device, the 2D MRI slices to a segmentation model to extract regions of interest (ROIs);
   inputting, by the analysis device, pixel information about the ROIs to a pre-trained first learning model to predict a cortical thickness of at least one region of the ROIs and a volume of at least one region of the ROIs; and
   inputting, by the analysis device, the cortical thickness of the at least one region and the volume of the at least one region to a pre-trained second learning model to output dementia information about the subject.
   wherein the ROIs include at least one of frontal gray matter, temporal gray matter, parietal gray matter, occipital gray matter, lateral ventricle (LV), hippocampus, and extracerebral cerebrospinal fluid.

2. The dementia information calculation method of claim 1, wherein the first learning models receives the summed number of pixels of the ROIs and clinical information about the subject to predict the cortical thickness and the volume, and the clinical information includes at least one of age, gender, and APOE4 genotype of the subj ect.

3. The dementia information calculation method of claim 1, wherein the first learning models further receive a brain size calculated from the 2D MRI slices to predict the cortical thickness and the volume.

4. The dementia information calculation method of claim 1, wherein the first learning model includes multiple learning models prepared in advance for each ROI.

5. The dementia information calculation method of claim 1, wherein the second learning model receives at least one of a cortical thickness of frontal gray matter, a cortical thickness of temporal gray matter, a cortical thickness of parietal gray matter, and a cortical thickness of occipital gray matter and receives at least one of a volume of the LV, a volume of the hippocampus, and a volume of the extracerebral cerebrospinal fluid to output the dementia information about the subject.

6. The dementia information calculation method of claim 5, wherein the second learning model further receives at least one information of the age, gender, and APOE4 genotype of the subject.

7. A dementia information calculation method using two-dimensional magnetic resonance imaging (MRI), comprising:

   receiving, by an analysis device, inputs of 2D MRI slices of a subject;
   inputting, by the analysis device, the 2D MRI slices to a segmentation model to extract regions of interest (ROIs);
   inputting, by the analysis device, pixel information of the ROIs to a pre-trained first learning model to predict a volume of at least one region of the ROIs; and
   inputting, by the analysis device, the volume of the at least one region to a pre-trained second learning model to output dementia information about the subject,
   wherein the ROIs include at least one of frontal gray matter, temporal gray matter, parietal gray matter, occipital gray matter, lateral ventricle (LV), hippocampus, and extracerebral cerebrospinal fluid.

8. The dementia information calculation method of claim 7, wherein the first learning models receive the summed number of pixels of the ROIs and clinical information about the subject to predict the volume, and the clinical information includes at least one of age, gender, and APOE4 genotype of the subject.

9. The dementia information calculation method of claim 7, wherein the first learning models receive a brain size calculated from the 2D MRI slices to predict the volume.

10. The dementia information calculation method of claim 7, wherein the first learning model includes multiple learning models prepared in advance for each ROI.

11. The dementia information calculation method of claim 7, wherein the second learning model receives at least one of a cortical thickness of frontal gray matter, a cortical thickness of temporal gray matter, a cortical thickness of parietal gray matter, and a cortical thickness of occipital gray matter and receives at least one of a volume of the LV, a volume of the hippocampus, and a volume of the extracerebral cerebrospinal fluid to output the dementia information about the subject.

12. The dementia information calculation method of claim 11, wherein the second learning model further receives at least one information of the age, gender, and APOE4 genotype of the subject.

13. An analysis device for calculating dementia information, the analysis device comprising:

an interface device that receives two-dimensional magnetic resonance imaging (2D MRI) slices of a subject;
a storage device configured to store a segmentation model that extracts a region of interest (ROI) from the 2D MRI slice, a first learning model that receives ROI information to predict a volume, and a second learning model that calculates dementia information; and
a calculation device configured to input the received 2D MRI slices into the segmentation model to extract ROIs, input pixel information of at least one first region of the extracted ROIs to the first learning model to predict a volume of the at least one first region, and input the volume of the at least one first region to the second learning model to output the dementia information about the subject,
wherein the ROIs include at least one of frontal gray matter, temporal gray matter, parietal gray matter, occipital gray matter, lateral ventricle (LV), hippocampus, and extracerebral cerebrospinal fluid.

14. The analysis device of claim 13, wherein the first learning model includes multiple learning models prepared in advance for each ROI.

15. The analysis device of claim 13, wherein the at least one first region includes the LV, the hippocampus, and the extracerebral cerebrospinal fluid.

16. The analysis device of claim 13, wherein the storage device further stores a third learning model that receives the ROI information to predict a cortical thickness, and
the calculation device inputs pixel information of at least one second region of the extracted ROIs to the third learning model to predict a cortical thickness of the at least one second region, and further inputs the cortical thickness of the at least one second region to the second learning model to output the dementia information about the subject.

17. The analysis device of claim 16, wherein the at least one second region includes at least one of frontal gray matter, temporal gray matter, parietal gray matter, and occipital gray matter.

18. The analysis device of claim 13, wherein the calculation device further inputs at least one information of age, gender, and APOE4 genotype of the subject to the second learning model to output the dementia information about the subject.

## FIG. 1

| 110 | 120 | 130 | 140 | 150 |
|---|---|---|---|---|
| 2D MRI | EXTRACT ROI | CALCULATE BRAIN IMAGE INDEX | PREDICT CORTICAL THICKNESS AND/ OR VOLUME OF ROI | OUTPUT DEMENTIA INFORMATION |

**100**

**FIG. 2**

**FIG. 3**

# FIG. 4

GROUND
TRUTH

410

420

EXTRACT
INPUT
DATA

INPUT DATA

VOLUME

LV

HIPPOCAMPUS

EXTRACEREBRAL
CEREBROSPINAL
FLUID

CLINICAL INFORMATION

AGE/GENDER/APOE4

LEARNING
MODEL

DEMENTIA
INFORMATION

400

TRAINING REPETITION

**FIG. 5**

GROUND
TRUTH

510

520

EXTRACT
INPUT
DATA

INPUT DATA

CORTICAL THICKNESS

FRONTAL

TEMPORAL

PARIETAL

OCCIPITAL

VOLUME

LV

HIPPOCAMPUS

EXTRACEREBRAL
CEREBROSPINAL
FLUID

CLINICAL INFORMATION

AGE/GENDER/APOE4

LEARNING
MODEL

DEMENTIA
INFORMATION

**500**

TRAINING REPETITION

# FIG. 6

ANALYSIS DEVICE

**600**

| STORAGE DEVICE (610) | INTERFACE DEVICE (640) |
|---|---|
| MEMORY (620) | COMMUNICATION DEVICE (650) |
| CALCULATION DEVICE (630) | OUTPUT DEVICE (660) |

## INTERNATIONAL SEARCH REPORT

| International application No. |
|---|
| **PCT/KR2023/001929** |

**A. CLASSIFICATION OF SUBJECT MATTER**

**A61B 5/00**(2006.01)i; **A61B 5/055**(2006.01)i; **G16H 50/20**(2018.01)i; **G06T 7/11**(2017.01)i; **G06T 7/00**(2006.01)i

According to International Patent Classification (IPC) or to both national classification and IPC

**B. FIELDS SEARCHED**

Minimum documentation searched (classification system followed by classification symbols)

A61B 5/00(2006.01); A61B 5/055(2006.01); A61B 6/00(2006.01); A61B 6/03(2006.01); G06F 19/00(2011.01); G16H 50/20(2018.01); G16H 50/30(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 치매(dementia), 분석(analysis), 자기공명(magnetic resonance, MR), 이미지(image), 분할(segmentation), 관심영역(region of interest), 학습(learning), 두께(thickness), 부피(volume)

**C. DOCUMENTS CONSIDERED TO BE RELEVANT**

| Category* | Citation of document, with indication, where appropriate, of the relevant passages | Relevant to claim No. |
|---|---|---|
| Y | KR 10-1929965 B1 (GACHON UNIVERSITY OF INDUSTRY-ACADEMIC COOPERATION FOUNDATION) 17 December 2018 (2018-12-17)<br>See paragraph [0077]; and claim 1. | 1-18 |
| Y | KR 10-2021-0029318 A (KOREA UNIVERSITY RESEARCH AND BUSINESS FOUNDATION et al.) 16 March 2021 (2021-03-16)<br>See paragraph [0053]; and claims 1 and 3. | 1-18 |
| A | KR 10-2020-0062589 A (THE ASAN FOUNDATION et al.) 04 June 2020 (2020-06-04)<br>See entire document. | 1-18 |
| A | US 2020-0027557 A1 (HUMAN LONGEVITY, INC.) 23 January 2020 (2020-01-23)<br>See entire document. | 1-18 |
| A | WO 2016-205811 A1 (NEW YORK UNIVERSITY) 22 December 2016 (2016-12-22)<br>See entire document. | 1-18 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 May 2023** | **30 May 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/001929**

| Patent document cited in search report | | | Publication date (day/month/year) | Patent family member(s) | | | Publication date (day/month/year) |
|---|---|---|---|---|---|---|---|
| KR | 10-1929965 | B1 | 17 December 2018 | None | | | |
| KR | 10-2021-0029318 | A | 16 March 2021 | EP | 4026498 | A2 | 13 July 2022 |
| | | | | EP | 4026498 | A4 | 29 March 2023 |
| | | | | JP | 2022-547909 | A | 16 November 2022 |
| | | | | KR | 10-2276545 | B1 | 15 July 2021 |
| | | | | US | 2022-0335611 | A1 | 20 October 2022 |
| | | | | WO | 2021-045507 | A2 | 11 March 2021 |
| | | | | WO | 2021-045507 | A3 | 29 April 2021 |
| KR | 10-2020-0062589 | A | 04 June 2020 | KR | 10-2250954 | B1 | 12 May 2021 |
| US | 2020-0027557 | A1 | 23 January 2020 | WO | 2019-169049 | A1 | 06 September 2019 |
| WO | 2016-205811 | A1 | 22 December 2016 | US | 10657642 | B2 | 19 May 2020 |
| | | | | US | 2019-0012783 | A1 | 10 January 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **REBSAMEN, M et al.** Direct Cortical Thickness Estimation Using Deep Learning-Based Anatomy Segmentation and Cortex Parcellation.. *Human Brain Mapping*, 2020, vol. 41 (17), 4804-4814
- **RENAUD LA JOIE et al.** Region-Specific Hierarchy Between Atrophy, Hypometabolism, and β-amyloid (Aβ) load in Alzheimer's Disease Dementia. *J. Neurosci.*, 14 November 2012, vol. 32 (46), 16265-73
- **JIN SAN LEE et al.** Machine Learning-based Individual Assessment of Cortical Atrophy Pattern in Alzheimer's Disease Spectrum: Development of the Classifier and Longitudinal Evaluation. *J Neurosci. SCIENTIFIC REPORTS*, 2018, vol. 8 (1), 4161